Europäisches Patentamt

European Patent Office

Office européen des brevets

(11) Publication number: **0 145 419**
**A2**

(12) **EUROPEAN PATENT APPLICATION**

(21) Application number: **84308303.1**

(22) Date of filing: **29.11.84**

(51) Int. Cl.⁴: **A 61 F 11/04,** H 03 H 7/00

(30) Priority: **09.12.83 US 559874**

(43) Date of publication of application: **19.06.85**
**Bulletin 85/25**

(84) Designated Contracting States: **AT CH DE FR GB LI**

(71) Applicant: **MINNESOTA MINING AND MANUFACTURING COMPANY, 3M Center, P.O. Box 33427, St. Paul, MN 55133 (US)**

(72) Inventor: **Fretz, Robert J. c/o Minnesota Mining and Manufact, uring Company 2501 Hudson Road P.O. Box 33427, St. Paul, Minnesota 55133-3427 (US)**

(74) Representative: **Baillie, Iain Cameron, c/o Ladas & Parry Isartorplatz 5, D-8000 München 2 (DE)**

(54) **Direct current blocking circuit and implantable device using same.**

(57) A direct current blocking circuit (18) adapted for use with an electrical load (14). First (12) and second (20) blocking devices are coupled in series with each other and in series with the electrical load (14) for substantially blocking the passage of direct current through the load (14). A shunt device (22) is coupled in parallel with the load (14) and coupled between the first (12) and second (20) blocking devices for shunting a portion of the direct current leaking through the first blocking device (12). In this manner, the load (14) is protected against the passage of direct current not only by the first blocking device (12) and the second blocking device (20) but also by the limited voltage appearing across the shunt device (22). Also provided is an implantable device for stimulating body tissue which utilizes the DC blocking circuit (18) described above.

## DIRECT CURRENT BLOCKING CIRCUIT AND
## IMPLANTABLE DEVICE USING SAME

### Technical Field

The present invention relates generally to direct current blocking circuits, and in one embodiment, more particularly to an implantable device utilizing a direct current blocking circuit.

### Background Art

Certain implantable electronic devices utilize an alternating current waveform for signal transmission. An example is an implantable hearing prosthesis in which an electrode is implanted in the ear, either in the cochlea or in the middle ear. Alternating current (AC) electrical signals are then delivered to the electrode to electrically stimulate the auditory nerve. In some such systems, the AC electrical signals are transmitted across the skin boundary by inductive coupling between an external coil and an internal coil. In some such systems, the direct current (DC) component of the AC electrical signal is substantial.

In such biomedical applications, it may be desirable for biological reasons to limit the DC component of the electrical signal which reaches the electrode.

Prior art systems have limited the DC component by inserting a DC blocking capacitor in series with the electrode (load). In theory, and with no defects, this approach is perfectly acceptable. However, most, if not all, electronic capacitors are not perfect capacitors and do not perfectly block direct current. The result is a DC leakage current through the capacitor. This DC leakage current could possibly increase to hazardous levels through a defective capacitor caused either by a manufacturing defect or as a result of the severe environment in which the capacitor resides, namely the body.

A straight forward approach of doubling or tripling the protection by merely adding additional

-2-    0145419

capacitors in series with the electrode to serve as additional direct current blocks is not desirable. Any leakage current through the first device will be passed to the next device which could also fail.

Summary of Invention

The present invention is a direct current blocking circuit adapted for use with an electrical load. A first blocking device is coupled in series with the load for substantially blocking the passage of direct current through the load. A second blocking device is coupled in series with the first blocking device and the load for substantially blocking the passage of direct current through the load. Further, a shunt device is coupled in parallel with the load and coupled between the first blocking device and the second blocking device for shunting a portion of any direct current leaking through the first blocking device. In this way, the load is protected against the passage of direct current not only by the first blocking device and the second blocking device but also by the limited voltage appearing across the shunt device. In a preferred embodiment, the value of the resistance of the shunt device is greater than, and, in a still preferred embodiment, is much greater than the value of the resistance of the load.

In another embodiment, the present invention is an implantable device having an electrical load. A first blocking device is coupled in series with the electrical load for substantially blocking the passage of direct current through the electrical load. A second blocking device is coupled in series with the first blocking device and the electrical load for substantially blocking the passage of direct current through the electrical load. Further, a shunt device is coupled in parallel with the electrical load and coupled between the first blocking device and the second blocking device for shunting a portion of any direct current leaking through the first blocking device. In this way the electrical load is protected against the passage of direct current not only by the first blocking device and the second blocking device but also by the limited voltage appearing across the shunt device. In a preferred embodiment, the value of the resistance of the shunt device is greater than, and, in a still preferred embodiment, is much greater than the value of the resistance of the load. In a preferred embodiment, the blocking devices are capacitors.

The combination of the shunt device and the additional blocking device with the first blocking device achieves a unique and unexpected result. If all components work perfectly, then no DC leakage current reaches the electrode, as would be the case in prior art systems. If the first blocking device passes a DC leakage current but the second blocking device operates perfectly, then, again, no DC leakage current reaches the electrode, as would be the case in prior art systems where the blocking devices were merely doubled. However, since few, if any, blocking devices are perfect or operate without failure, the important issue is to investigate the operation of the circuit when both blocking devices are imperfect or fail. With prior art systems any DC leakage current would be passed directly to the load (electrode).

With the systems of the present invention, normal AC operation occurs. The AC signal is delivered to the electrode through the current division effect of the parallel combination of the shunt resistance and the electrode resistance. If both blocking devices pass a DC leakage current, only a very small portion of that leakage current would reach the electrode. The DC leakage current passing through the first blocking device would encounter alternate paths, either through the shunt device or through the second blocking device. Even if the second blocking device is also passing a DC leakage current, its impedance would still be sufficiently high that most of the DC leakage current from the first blocking device would pass through the shunt device.

Thus, the present invention provides a unique and unexpected result of protecting against significant levels of DC leakage current passing through the load, even with imperfect or failed DC blocking devices.

## Brief Description of Drawings

The foregoing advantages, construction and operation of the present invention will become more readily apparent from the following description and accompanying drawings in which:

Figure 1 is a block diagram of an exemplary prior art system;

Figure 2 is a block diagram of the system of the present invention;

Figure 3 is a detailed circuit diagram of the system of the present invention;

Figure 4 is an alternative detailed circuit diagram of the system of the present invention;

Figure 5 is a circuit diagram of a prior art implantable device; and

Figure 6 is a circuit diagram of an implantable device using the present invention.

## Detailed Description

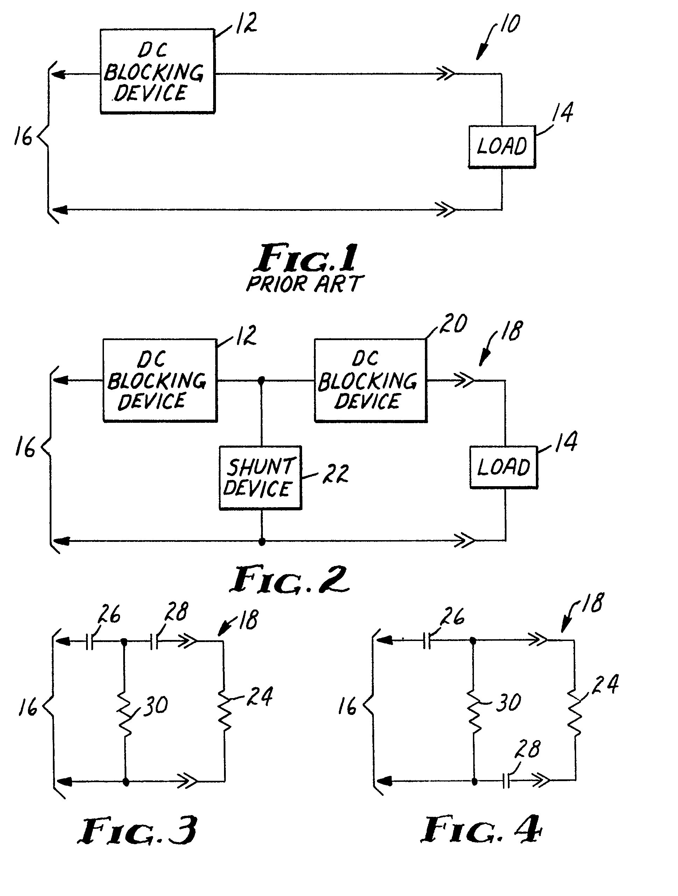

Figure 1 is a block diagram of an exemplary system 10 utilizing a DC blocking circuit. A DC blocking device 12 is coupled in series with a load 14 which in turn is coupled to an electrical supply 16. The electrical supply 16 is intended primarily to transmit an alternating current (AC) signal for transmission through the DC blocking device 12 to the load 14. However, the electrical supply 16 also contains an undesirable direct current (DC) component. In the system 10 of Figure 1, all of the direct current component will be blocked by the DC blocking device 12 provided that the DC blocking device 12 works perfectly. However, should the DC blocking device not be a perfect DC block, or should the DC blocking device 12 fail, all of the DC leakage current through the DC blocking device 12 would pass through load 14. It does little good to double up on the DC blocking devices 12 by inserting another in series since any leakage or any cumulative failure of the DC blocking devices 12 would result in the DC leakage current passing through all DC blocking devices 12 reaching the load 14.

Figure 2 illustrates the system 18 of the present invention. Again, a load 14 is ultimately connected to an electrical supply 16. Again, a DC blocking device 12 is coupled in series with the load 14 in order to block the undesirable direct current component of the electrical supply 16. However, in Figure 2 an additional DC blocking device 20 is coupled in series with the load 14 and a shunt device 22 is coupled in parallel with the load 14 and coupled between DC blocking device 12 and DC blocking device 20.

The effect of such a circuit arrangement is significant. With respect to the alternating current component of the electrical supply 16, a portion of the AC alternating current supply is delivered to the load 14. Shunt device 22 and load 14 combine to form a divider in which a portion of the total alternating current available from the electical supply 16 will pass through the load in the inverse proportion of their respective impedances. If the impedance of shunt device 22 is significantly higher than the impedance of load 14, most of the alternating current signal available from the electrical supply 16 will be delivered to load 14. Even if the impedance of shunt device 22 is equal to the impedance of load 14, a full one half of the AC signal available from the electrical supply 16 will pass through load 14.

Since few, if any, DC blocking devices 12 and 20 are perfect DC blocks, or even if they are perfect, they are subject to failure in use. Some DC leakage current can be expected at some point in time through both DC blocking device 12 and DC blocking device 20. However, only a small portion of the DC leakage current, which in prior art systems would otherwise flow through load 14, ultimately reaches the load 14. Hence, the DC leakage current passing through load 14 can be prevented from reaching hazardous levels.

The DC leakage current passing through DC blocking device 12 encounters alternate paths. The DC blockage current passing through DC blocking device 12 can either pass through shunt device 22 or through DC blocking device 20 and load 14. Even if DC blocking device 20 is passing a significant level of DC leakage current, its impedance would still be sufficiently high that most of the DC leakage current from DC blocking device 12 would pass through shunt device 22, thus avoiding load 14.

This effect can be more readily illustrated by reference to Figure 3 which is a detailed circuit diagram of a preferred embodiment of the system 18 of the present invention. Again, an electrical supply 16 is coupled to the system 18. The load is represented by resistor 24. Capacitors 26 and 28 form the first DC locking device 12 and the second blocking device 20, respectively. Capacitors 26 and 28 are coupled in series and in themselves are coupled in series with resistor 24. Resistor 30 is coupled in parallel with resistor 24 and is coupled between capacitors 26 and 28 and represents the shunt device 22.

In a typical example of a cochlear implant, the voltage available from electrical supply 16 would be approximately five volts DC, i.e. the undesirable component of electrical supply 16. The load resistance from resistor 24, representing the electrode, would be approximately 1,000 ohms. Assume that the capacitors 26 and 28 each would have a leakage resistance of five megohms, which is very leaky for a high grade capacitor. With a DC blocking circuit as in the prior art, the amount of DC leakage current passing through resistor 24 would simply be the DC voltage component of five volts divided by the leakage resistance of the capacitor of five megohms plus the resistance of the load of 1,000 ohms, or approximately 5 megohms. Thus, the leakage current reaching the load would be approximately 1 microampere. With the circuit of the present invention in Figure 3, assuming that both capacitors 26 and 28 have a DC leakage resistance of five megohms and that shunt resistor 30 has a resistance of 30 kilohms, the voltage appearing across shunt resistor 30 would be five volts times the ratio of the resistance of the shunt resistor 30 with the leakage resistance of capacitor 26 or, five volts times the quantity 30 kilohms divided by five megohms, or 0.03 volts. With the DC leakage resistance of capacitor 28 also being five megohms, the leakage occurring through resistor 24 would be the voltage across resistor 30 of 0.03 volts divided by the combination of the resistance of capacitor 28 along with resistor 24, or approximately five megohms. This would result in a leakage current through resistor 24 of 0.006 microamperes. Thus, the system of the present invention involving the DC blocking circuit and the implantable device utilizing the DC blocking circuit results in reducing the level of DC current reaching the load by over two orders of magnitude. This contrasts with the result obtained by merely doubling capacitor 26 and 28 without including shunt resistance 30 which is a reduction in the leakage current merely by a factor of two.

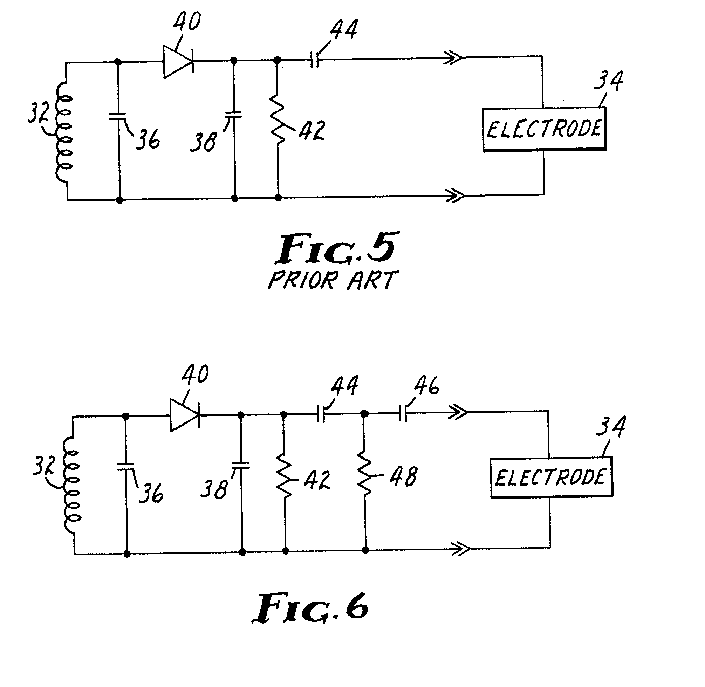

Figure 4 illustrates an alternative circuit diagram of the system 18 of the present invention. The circuit is similar to the circuit in Figure 3 except that capacitor 28 has been moved below shunt resistor 30 and load resistance 24. Otherwise, operation of the circuit is identical. Again, it is noted that capacitors 26 and 28 are coupled in series with each other and in series with load resistor 24 and that shunt resistance 30 is coupled in parallel with the load resistance 24 and between capacitors 26 and 28.

Figure 5 shows an exemplary implantable device. Coil 32 is implanted under the skin to receive by inductive coupling the AC signal to be transmitted to the electrode 34. Capacitors 36 and 38, diode 40 and resistor 42 combine to provide a circuit which drives the electrode 34 with the AC signal inductively coupled to coil 32. Unfortunately, the signal also contains an undesirable DC component, hence, capacitor 44 is added in series to the electrode 34 to block that DC component. This circuit suffers from the same problem discussed with prior art circuit in Figure 1.

Figure 6 illustrates a schematic diagram of an implantable device utilizing the protective DC blocking circuit of the present invention. Again, coil 32, electrode 34, capacitor 36 and 38, diode 40 and resistor 42 operate conventionally as in Figure 5. Instead of the single DC blocking capacitor 24, the device of Figure 6 has a second DC blocking capacitor 46 coupled in series with capacitor 44 and in series with electrode 34. Further, the device of Figure 6 has a shunt resistance 48 coupled in parallel with electrode 34 and between capacitors 44 and 46. In operation with exemplary values as described with respect to Figure 3, the device of Figure 6 lowers the potential DC leakage current through the electrode 34 by over two orders of magnitude.

Of course, it is to be recognized and understood that in Figures 1 through 6 the load or electrode has been illustrated as being connectable to the circuitry and that in other environments, one or more of the other components of the electronic circuit could be packaged and coupled with the load or the electrode without adverse effect on circuit operation.

Thus, it can be seen that there has been shown and described a novel direct current blocking circuit and implantable device using same. It is to be understood that various changes, modifications and substitutions in the form and details of the described circuit and device can be made by those skilled in the art without departing from the scope of the invention as described by the following claims.

0145419

CLAIMS:

1. A direct current blocking circuit (18) adapted for use with an electrical load (14), having first blocking means (12) coupled in series with said load (14) for substantially blocking the passage of direct current through said load (14); characterized by further having second blocking means (20) coupled in series with said first blocking means (12) and said load (14) for substantially blocking the passage of direct current through said load (14); and shunt means (22) coupled in parallel with said load (14) and coupled between said first blocking means (12) and said second blocking means (20) for shunting a portion of any direct current leaking through said first blocking means (12); whereby said load (14) is protected against the passage of direct current not only by said first blocking means (12) and said second blocking means (20) but also by the limited voltage appearing across said shunt means (22).

2. A direct current blocking circuit (18) as in Claim 1 wherein the impedance of said load (14) is primarily resistive.

3. A direct current blocking circuit (18) as in Claim 2 wherein the value of the resistance of said shunt means (22) is greater than the value of the resistance of said load (14).

4. A direct current blocking circuit (18) as in Claim 2 wherein the value of the resistance of said shunt means (22) is much greater than the value of the resistance of said load (14).

5. A direct current blocking circuit (18) as in Claim 3 wherein the impedance of said first blocking means (12) is primarily capacitive and wherein the impedance of said second blocking means (20) is primarily capacitive.

6. A direct current blocking circuit (18) as in Claim 5 wherein said first blocking means (12) is a capacitor and wherein said second blocking means (20) is a capacitor.

7. A direct current blocking circuit (18) as in Claim 6 wherein the impedance of said shunt means (22) is primarily resistive.

8. An implantable device adapted to stimulate body tissue, comprising:
first blocking means (44) adapted to be coupled in series with said body tissue for substantially blocking the passage of direct current through said body tissue;
second blocking means (46) coupled in series with said first blocking means (44) for substantially blocking the passage of direct current through said body tissue; and
shunt means (48) adapted to be coupled in parallel with said body tissue and coupled between said first blocking means (44) and said second blocking means (46) for shunting a portion of any direct current leaking through said first blocking means (44);
whereby said body tissue is protected against the passage of direct current not only by said first blocking means (44) and said second blocking means (46) but also by the limited voltage appearing across said shunt means (48).

0145419

9. An implantable device as in Claim 8 wherein said first blocking means (44) is a capacitor and wherein said second blocking means (46) is a capacitor.

10. An implantable device as in Claim 9 wherein the impedance of said shunt means (48) is primarily resistive.

0145419

112

FIG.1
PRIOR ART

FIG.2

FIG.3

FIG.4

0145419

2/2

FIG.5
PRIOR ART

FIG.6